(19) Europäisches Patentamt
European Patent Office
Office européen des brevets

(11) Publication number: **0 058 070**
**A2**

# (12) EUROPEAN PATENT APPLICATION

(21) Application number: 82300603.6

(22) Date of filing: 08.02.82

(51) Int. Cl.³: C 07 D 213/65
//C07D213/89, C07D405/04

(30) Priority: 09.02.81 US 232923

(43) Date of publication of application:
18.08.82 Bulletin 82/33

(84) Designated Contracting States:
AT BE CH DE FR GB IT LI LU NL SE

(71) Applicant: PFIZER INC.
235 East 42nd Street
New York, N.Y. 10017(US)

(72) Inventor: Cue, Jr. Berkeley Wendell
266 Stoddards Wharf Drive
Gales Ferry Connecticut(US)

(72) Inventor: Massett, Stephen Sargent
78 Brandegee Avenue
Groton Connecticut(US)

(74) Representative: Wood, David John et al,
Pfizer Limited Ramsgate Road
Sandwich Kent CT13 9NJ(GB)

## (54) Process for preparing pirbuterol.

(57) Pirbuterol is prepared by heating 5-hydroxy-2-(1-hydroxy-2-*t*-butylaminoethyl)pyridine with aqueous formaldehyde in the presence of a weak base, e.g. triethylamine. The pirbuterol is conveniently isolated as the hydrochloride. Pirbuterol is a well known bronchodilator.

EP 0 058 070 A2

Croydon Printing Company Ltd.

# PROCESS FOR PREPARING PIRBUTEROL

This invention relates to a process for the preparation of pirbuterol (or a salt thereof) which has the formula:-

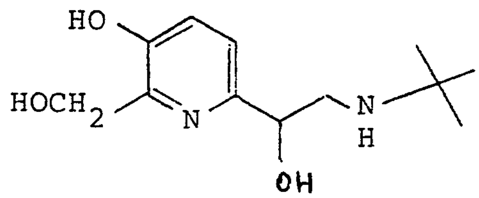

(I).

Pirbuterol and its bronchodilator activity were originally described by Barth in a series of U.S. Patents (3,700,681; 3,763,173; 3,772,314; 3,786,160). At that time, pirbuterol was synthesized as follows:

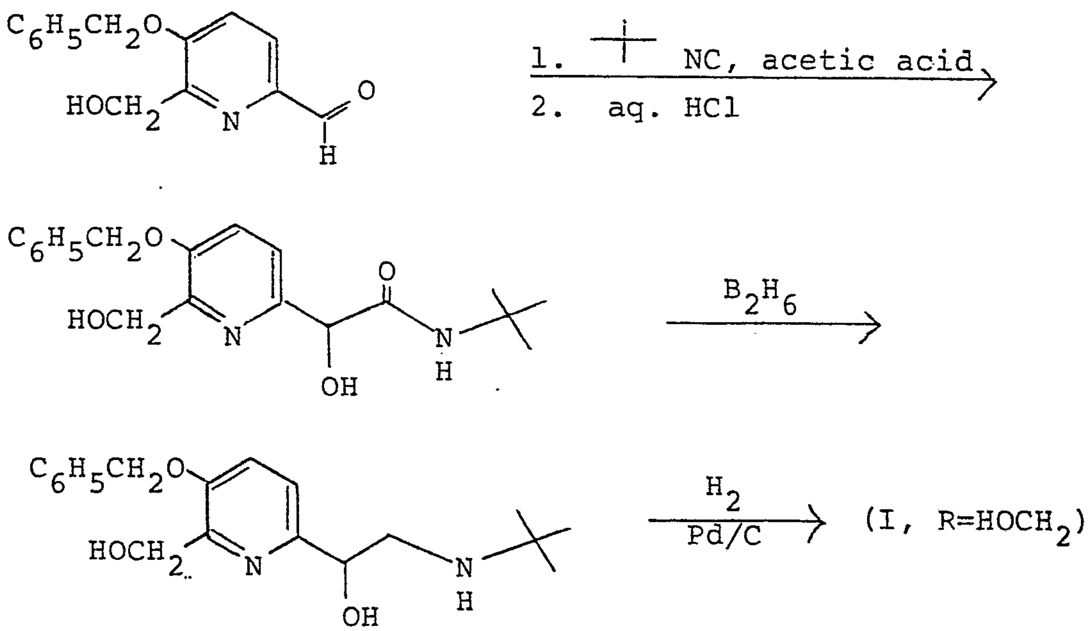

Pirbuterol analogs wherein the hydroxymethyl group is replaced by hydrogen, methyl, or methanesulfonylmethyl, and their bronchodilator activity, were disclosed by Jen and Kaiser in U.S. Patent 3,952,101. These compounds were prepared from the corresponding aldehydes by a reaction sequence fully analogous with that of the earlier pirbuterol process.

Subsequently, an alternative, preferred process for the synthesis of pirbuterol was described by Nakanishi (U.S. Patents 3,948,919; 4,031,108), exemplified as follows:

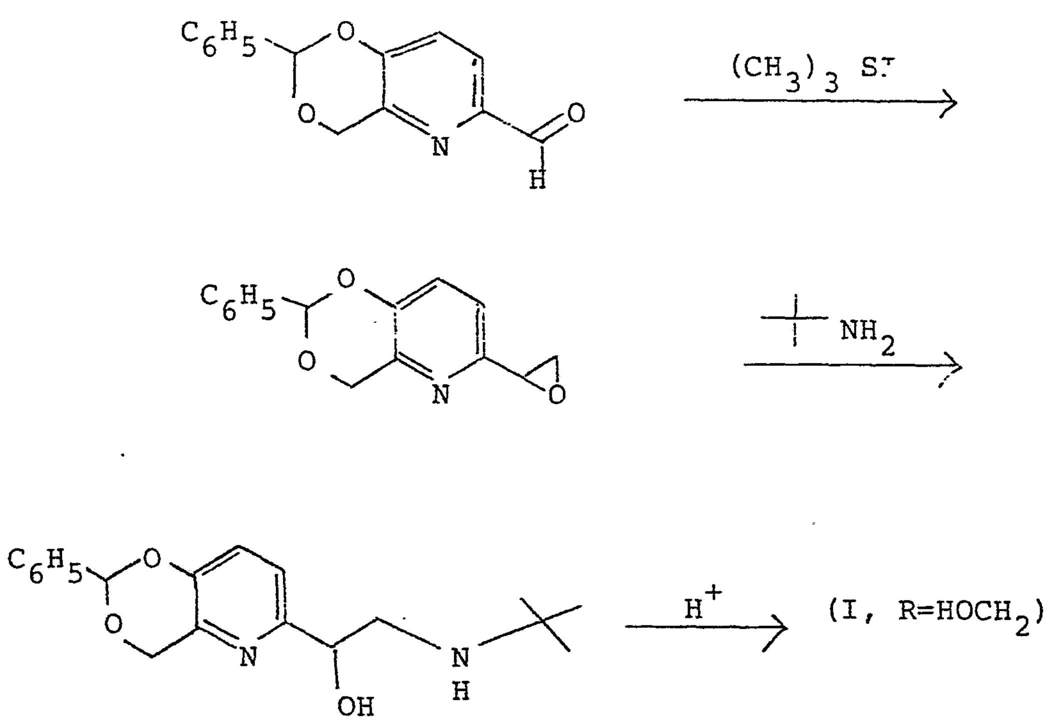


Improvements and alternatives to this process have also been described, viz., isolation of the pirbuterol precursor as a maleate salt (Carroll et al., U.S. Patent 4,011,231) and hydrogenolysis rather than hydrolysis as the final stage (Argentina Patent 214005, Luxembourg Patent 79564).

The latter process still suffers from the disadvanatge of generating noxious sulfide by-products in the preparation of epoxide. Even under the best of conditions, traces of sulfur can carry through and increase the catalyst level when protecting groups are removed by hydrogenolysis, the preferred route when the free base form of pirbuterol is desired. Furthermore, the epoxide is relatively unreactive towards the tert-butylamine reagent, even under pressure at elevated temperatures requiring large excess of the reagent and a relatively long time to achieve complete reaction.

A further disadvantage of the improved process is the polar nature of intermediate amino alcohol, making this intermediate difficult to isolate in pure form.

Recently pirbuterol has been discovered to also have utility for the treatment of congestive heart failure (Taylor, U.S. Patent 4,175,128).

In a chemical transformation related to one of the process steps of the present invention, Benderly et al., [Can. J. Chem. 56, pp. 2673-2676 (1978)] have reported the conversion of 2-vinylpyridine to 2-(1,2-epoxyethyl)-pyridine N-oxide by the action of m-chloroperbenzoic acid on 2-vinylpyridine.

The present invention provides a method for the synthesis of pirbuterol, involving hydroxymethylation, as follows:

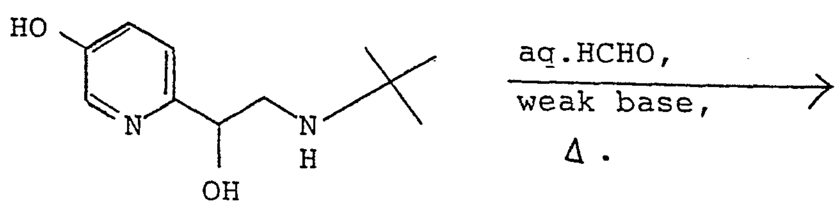


(IA)

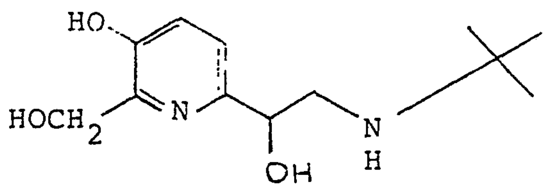

(I)

The pirbuterol is conveniently isolated as a salt (e.g. the hydrochloride).

This process permits a single step synthesis from a precursor having potential commercial availability by dint of its own biological activity.

The hydroxymethylation of the compound (IA) to yield pirbuterol (I) is typically carried out by heating the former in excess aqueous formaldehyde in the presence of an excess of a weak base. Reaction temperatures in the range 85-105°C. are generally used, the reflux temperature of the reaction mixture being well-suited. The weak base should be one which does not react to any significant degree with starting materials, or product, or cause a high degree of polymerization of the formaldehyde. Tertiary aliphatic amines, conveniently the readily available and inexpensive triethylamine, are well suited as the weak base catalyst for this reaction.

The starting material is available by conventional methods, e.g. those detailed in the Jen and Kaiser U.S. Patent cited above.

The present invention is illustrated by the following Example :-

EXAMPLE

Pirbuterol Hydrochloride

A solution of 5-hydroxy-2-(1-hydroxy-2-*tert*-butylaminoethyl)pyridine (0.500 g., 0.00238 mole) in 5 ml. of aqueous 38% formaldehyde solution and 1 ml. of triethylamine was heated at the reflux temperature overnight. An additional 5 ml. of aqueous formaldehyde solution was added and heating at the reflux temperature was continued for 6 hours. The solvents were evaporated *in vacuo* to give the crude product which was isolated by dissolving it in a minimum volume of cold methanol and saturating the cold solution with dry hydrogen chloride to give 0.52 g. (70%) of pirbuterol hydrochloride m.p. 174-176° (dec.).

The following Preparations illustrate the Preparation of the intermediate used in the Example:-

PREPARATION 1

A. 5-Benzyloxy-2-vinylpyridine

In a two liter 3-neck round bottom flask fitted with a mechanical stirrer, thermometer and a water-cooled reflux condenser was placed 23.4 g. (0.11 mole) of 5-benzyloxypyridine-2-carbaldehyde, 78.5 g. (0.22 mole) of methyltriphenylphosphonium bromide and 26.5 g. (0.66 mole) of sodium hydroxide pellets in 250 ml. of toluene and 665 ml. of water. The reaction mixture was stirred at room temperature for 15 hours. The toluene layer was separated and saved. The aqueous layer was washed with toluene and the combined toluene layers are filtered through a 4" x 6" pad of silica gel to remove triphenylphosphate. The pad was successively washed with toluene, then ether/hexane (3:2) to remove the desired product. The organic solutions were combined, dried over anhydrous magnesium sulfate, filtered and evaporated *in vacuo* to give 5-benzyloxy-2-vinylpyridine as a yellow oil (20.5 g., 73.7%); NMR ($CDCl_3$) 8.15 (m, 1H, $H_6$), 7.16 (s, 5H, $C_6H_5$-), 7.00 (m, 2H, $H_3$ and $H_4$), 6.67 (d of d, 1H, olefin H), 5.95 (d of d, 1H, olefin H), 5.15 (d of d, 1H, olefin H) and 4.90 (3, 2H, $CH_2$).

B. 5-Benzyloxy-2-(1,2-epoxyethyl)pyridine N-Oxide

In a 3-neck 100 ml. round bottom flask fitted with a thermometer, magnetic stirrer/stirring bar and reflux condenser is placed 1.00 g. (0.005 mole) of 5-benzyloxy-2-vinylpyridine in 30 ml. of methylene chloride. To this vigorously stirred solution m-chloroperoxybenzoic acid (2.00 g., 0.01 mole) was added in one portion. The resulting solution was heated to reflux for 12 hours. Workup in the same manner as Example 2 gave 5-benzyloxy-2-(1,2-epoxyethyl)pyridine N-oxide [0.50 g.,(42%)]; m.p. 108-110°C. (dec.); NMR ($CDCl_3$) delta 8.00 (d, 1H, $H_6$), 7.33 (m, 5H, phenyl H), 7.30-7.10 (m, 2H, $H_3$ and $H_4$), 5.05 (s, 2H, $CH_2$ of benzyl), 4.47 (m, 1H, epoxide CH), 3.23 (d of d, 1H, epoxide CH) and 2.67 (m, 1H, epoxide H), mass spectrum (70 eV) m/e 243 ($M^+$), 152 ($M^+-CH_2C_6H_5$), 91 ($C_7H_7^+$).

C. 5-Benzyloxy-2-(1-hydroxy-2-*tert*-butylaminoethyl)-pyridine N-oxide

To 0.50 g. (0.0042 mole) of [5-benzyloxy-2-(1,2-epoxyethyl)pyridine N-oxide] in 20 ml. of methanol was added 0.5 ml. *tert*-butylamine and the resulting mixture heated to the reflux temperature for 4 hours. Solvent and excess *tert*-butylamine were removed *in vacuo* to give 280 mg. (43%) of the desired product after recrystallization from ether/ethyl acetate; m.p. 148-150° (dec.). Mass spec (70 eV) *m/e* 300 (M-16), *m/e* 243 [M-$H_2NC(CH_3)_3$); NMR (DMSO $d_6$)delta 7.90 (d, 1H, $H_6$), 7.30 (m, 6H, benzyl $C_6H_5$ and $H_4$), 7.00 (m, 1H, $H_3$), 5.07 (m, 3H, $\phi C\underline{H}_2$ and $\underset{\text{OH}}{\underset{|}{C}}\underline{H}CH_2$); 4.33 (m, 2H, $-\underset{\text{OH}}{\underset{|}{C}}H-C\underline{H}_2N$) and 1.13 [s, 9H, $C(C\underline{H}_3)_3$]; ir (KBr) 1171 ($N^+-O^-$).

D. 5-Hydroxy-2-(1-hydroxy-2-*tert*-butylaminoethyl)-pyridine

Into a 250 ml. Parr bottle there were introduced 250 mg. (0.79 mmole) of 5-benzyloxy-2-(1-hydroxy-2-*tert*-butylaminoethyl)pyridine N-oxide and 125 mg. of 5% palladium on carbon in 25 ml. of methanol. Under a hydrogen pressure of 45 psi, the mixture was shaken at room temperature for 6 hours. The catalyst was removed by filtration under a nitrogen atmosphere followed by removel of the solvent *in vacuo* to give the product which was triturated with isopropanol and filtered. In this manner, there was obtained 175 mg. (75%) of 5-hydroxy-2-(1-hydroxy-2-*tert*-butylamino-ethyl)pyridine as white crystals, m.p. 165-168°C. (decomp).

## PREPARATION 2

### A. 5-Benzyloxy-2-(1,2-epoxyethyl)pyridine

In a 500 ml. 3-neck round bottom flask fitted with a thermometer and a mechanical stirrer, there was introduced 26.7 g. (0.13 mole) of trimethylsulfonium iodide in 175 ml. of N,N-dimethylformamide. The stirred solution was cooled to 0-5°C. in an ice bath and 21.3 g. (0.39 mole) of sodium methoxide was added in one portion. After stirring the reaction mixture for 10 minutes at 0° there was added portionwise over a 15 minute period 21.3 g. (0.1 mole) of 5-benzyloxypyridine-2-carbaldehyde. When the addition was completed the reaction mixture was stirred at 0°C. for 15 minutes, then poured into ice cold water (1 liter). The aqueous solution was extracted with two 250 ml. portions of ether. The ether layers were separated, combined and washed with two 250 ml. portions of water, then dried over anhydrous magnesium sulfate, filtered and evaporated *in vacuo* to give an oil which rapidly crystallized to afford 20.7 g. (91%) of 5-benzyloxy-2-(1,2-epoxyethyl)pyridine, m.p. 45-47.5°C. (recrystallized from hexanes).

B. 5-Benzyloxy-2-(1-hydroxy-2-*tert*-butylaminoethyl)-pyridine

To a solution of 20.7 g. (0.091 mole) of 5-benzyloxy-2-(1,2-epoxyethyl)pyridine in 250 ml. of methanol, 25 ml. of *tert*-butylamine was added and the resulting solution was heated at the reflux temperature for 2.5 hours. The solvent and the excess *tert*-butylamine were evaporated *in vacuo* to give after recrystallization from hexanes, 17.3 g. (61%) of 5-benzyloxy-2-(1-hydroxy-2-*tert*-butylaminoethyl)pyridine; m.p. 69-73°C.

| | | | | |
|---|---|---|---|---|
| Anal. Calcd. for $C_{18}H_{24}O_2N_2$: | C, 71.97; | H, 8.05; | N, 9.33 |
| Found : | C, 71.86; | H, 7.96; | N, 9.26 |

C. 5-Hydroxy-2-(1-hydroxy-2-*tert*-butylaminoethyl)-pyridine

By the procedure of Preparation 1(D) 5-benzyloxy-2-(1-hydroxy-2-*tert*-butylaminoethyl)pyridine is hydrogenolyzed to produce 5-hydroxy-2-(1-hydroxy-2-*tert*-butylaminoethylpyridine.

CLAIMS

1. A process for the preparation of pirbuterol or an acid addition salt thereof, which comprises heating a compound of the formula

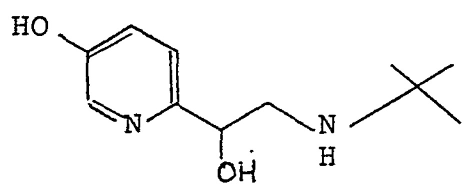

in aqueous formaldehyde in the presence of a weak base so as to form pirbuterol, followed by, optionally, conversion of the pirbuterol to an acid addition salt by reaction with a suitable acid.

2. A process according to claim 1, wherein the weak base is triethylamine.

3. A process according to claim 1 or 2, wherein an excess of aqueous formaldehyde and an excess of the weak base are used.

4. A process according to any one of the preceding claims, which is carried out at a temperature of from 85-105°C.